# EUROPÄISCHE PATENTSCHRIFT

(11) **EP 0 851 937 B1**
(45) Veröffentlichungstag und Bekanntmachung des Hinweises auf die Patenterteilung: **03.04.2002**
(21) Anmeldenummer: 96928466.0
(22) Anmeldetag: 14.08.1996
(51) Int. Cl.: C12Q 1/68

(54) **VERFAHREN ZUR REINIGUNG, STABILISIERUNG ODER ISOLIERUNG VON NUKLEINSÄUREN AUS BIOLOGISCHEN MATERIALIEN**
PROCESS FOR PURIFYING, STABILISING OR ISOLATING NUCLEIC ACIDS FROM BIOLOGICAL MATERIALS
PROCEDE DE PURIFICATION, DE STABILISATION, D'ISOLATION D'ACIDES NUCLEIQUES PROVENANT DE MATERIAUX BIOLOGIQUES

(30) Priorität: 16.08.1995 DE 19530132
(43) Veröffentlichungstag der Anmeldung: 08.07.1998
(73) Patentinhaber: Max-Planck-Gesellschaft zur Förderung der Wissenschaften e.V. Berlin, 80539 München (DE)
(72) Erfinder: MÜLLER, Oliver, D-44139 Dortmund (DE); DEUTER, Rainer, D-44309 Dortmund (DE)
(74) Vertreter: Weiss, Wolfgang, Dipl.-Chem. Dr.
(86) Internationale Anmeldenummer: EP9603595
(87) Internationale Veröffentlichungsnummer: WO9707239

(56) Entgegenhaltungen:
- EP-A- 0 189 280
- EP-A- 0 389 063
- EP-A- 0 393 744
- EP-A- 0 574 267
- WO-A-93/20235
- NUCLEIC ACIDS RESEARCH, Bd. 23, Nr. 18, 25.September 1995, Seiten 3800-3801, XP002017414 DEUTER ET AL.: "A METHOD FOR PREPARATION OF FECAL DNA SUITABLE FOR PCR"

## Beschreibung

Die vorliegende Erfindung betrifft ein Verfahren zur Stabilisierung, Reinigung oder/und Isolierung von Nukleinsäuren aus biologischen Materialien durch Entfernung von Verunreinigungen, z.B. von Nukleinsäuren schädigenden und enzymatische Reaktionen hemmenden Substanzen. Dieses Verfahren ist insbesondere zur Analyse, zum Nachweis oder zur Isolierung von Nukleinsäuren in Stuhlproben geeignet. Weiterhin wird ein für die Durchführung des erfindungsgemäßen Verfahrens geeignetes Reagenzienkit offenbart.

Zahlreiche Beispiele aus verschiedenen Forschungsbereichen belegen die Bedeutung der Analyse von Nukleinsäuren aus biologischen Materialien, die mit Substanzen verunreinigt sind, welche Nukleinsäuren während der Lagerung schädigen und eine enzymatische Manipulation der Nukleinsäuren, z.B. durch Amplifikation hemmen. Daher ist es für die Brauchbarkeit der in den biologischen Materialien enthaltenen Nukleinsäuren für weitere Analysen wichtig, daß diese Substanzen nur in sehr niedrigen Konzentrationen vorhanden sind oder gänzlich aus der Probe entfernt werden.

EP-A-0 393 744 offenbart ein Verfahren zur Extraktion einer Nukleinsäure aus einer wässrigen Vollblutprobe, wobei das Verfahren aus einem Erhitzen der Blutprobe am oder nahe dem Siedepunkt von Wasser für 2 bis 15 Minuten umfasst, um die Zellen in der Probe zu lysieren und die Nukleinsäure aus den Zellen freizusetzen, und dem Gewinnen der Nukleinsäure aus der erhitzten Probe besteht. Gegebenenfalls können der Blutprobe ein oder mehrere gelöste Polysaccharide in einer verdünnten wässrigen Salzlösung zugesetzt werden.

Eine besondere Bedeutung besitzt die Analyse von Nukleinsäuren aus fäkalen Proben. Die medizinische Hauptanwendung ist der Nachweis tumorspezifischer Veränderungen von nukleärer DNA aus Stuhl, die als Parameter bei der Frühdiagnose von Tumoren des Verdauungstrakts dienen können. Ebenso gewinnt der Nachweis bakterieller und viraler Infektionserreger aus Stuhlproben durch auf Nukleinsäuren basierende Testverfahren zunehmend an Bedeutung.

Eine Methode zur Isolierung von Nukleinsäuren aus Stuhl wird in WO 93/20235 offenbart. Diese Methode ergibt jedoch nur geringe Ausbeuten an Nukleinsäuren. Weiterhin werden DNA-schädigende oder/und PCR-hemmende Substanzen nicht abgetrennt. Daher kann die isolierte DNA nicht über längere Zeit gelagert werden und eine Amplifikation zur Vermehrung spezifischer, zu analysierender Genabschnitte dieser DNA liefert keine reproduzierbaren Ergebnisse. Ein besonders schwerwiegender Nachteil des bekannten Verfahrens besteht darin, daß bei der PCR-Amplifikation keine intakten DNA-Fragmente mit einheitlicher Sequenz entstehen, die für eine weitere Analyse notwendig sind. Um diese zu erhalten, ist eine aufwendige Klonierung der amplifizierten Genabschnitte notwendig. Noch ein weiterer Nachteil des Verfahrens des Standes der Technik besteht darin, daß die stark gesundheitsschädigenden Lösungsmittel Phenol und Chloroform verwendet werden müssen.

Eine der vorliegenden Erfindung zugrunde liegenden Aufgabe war somit die Bereitstellung eines Verfahrens, mit dem Nukleinsäuren in biologischen Materialien gegen Abbau stabilisiert und bei dem die enzymatische Manipulation von Nukleinsäuren hemmende Substanzen abgetrennt werden können. Insbesondere soll ein Verfahren bereitgestellt werden, das eine zuverlässige Isolierung von Nukleinsäuren aus fäkalen Proben ermöglicht.

Diese Aufgabe wird gelöst durch ein Verfahren zur Reinigung, Stabilisierung oder/und Isolierung von Nukleinsäuren aus biologischen Materialien, welches dadurch gekennzeichnet ist, daß man einer Nukleinsäuren enthaltenden Probe aus biologischen Materialien eine unlösliche Adsorptionsmatrix zur Bindung von Verunreinigungen zusetzt, die derart beschaffen ist, daß sie Verunreinigungen, die zur Schädigung von Nukleinsäuren führen oder/und die Durchführung enzymatischer Reaktionen verhindern oder/und enzymatische Reaktionen hemmen, binden kann und anschließend die Nukleinsäuren gegebenenfalls von gebundenen Verunreinigungen abtrennt. Das Inkontaktbringen der Nukleinsäuren enthaltenden Probe mit der Adsorptionsmatrix kann direkt oder nach Aufnehmen der Probe in Flüssigkeit erfolgen.

Durch das erfindungsgemäße Verfahren kann die Brauchbarkeit von aus biologischen Materialien isolierten Nukleinsäuren, insbesondere DNA, deutlich verbessert werden. Weiterhin werden durch den Zusatz der Adsorptionsmatrix sowohl Nukleinsäuren schädigende als auch die enzymatische Manipulation hemmende Substanzen weitgehend abgetrennt. Die durch das erfindungsgemäße Verfahren stabilisierten Nukleinsäuren können daher über längere Zeit gelagert werden. Darüber hinaus werden bei einer Amplifikation der durch Zusatz einer Adsorptionsmatrix behandelten Nukleinsäuren, z.B. durch PCR, reproduzierbare Ergebnisse erhalten. Diese Reproduzierbarkeit ist essentiell für die Aussagekraft der erhaltenen Ergebnisse bei der Nukleinsäure-Analyse. Die hohe Qualität der durch das erfindungsgemäße Verfahren gereinigter Nukleinsäuren zeigt sich beispielsweise darin, daß sie direkt durch Sequenzierung oder Heteroduplexanalyse untersucht werden können. Eine Klonierung ist nicht notwendig. Außerdem müssen beim erfindungsgemäßen Verfahren keine gesundheitsschädigenden Lösungsmittel eingesetzt werden.

Die beim erfindungsgemäßen Verfahren verwendete Adsorptionsmatrix ist derart beschaffen, daß sie Verunreinigungen, die zur Schädigung von Nukleinsäuren führen oder/und die Durchführung enzymatischer Reaktionen verhindern oder/und enzymatische Reaktionen hemmen, wie etwa Abbauprodukte von Hämoglobin, z.B. Bilirubin und dessen Abbauprodukte, oder/und Gallensäuren oder Salze davon oder deren Abbauprodukte sowie andere Abbauprodukte pflanzlichen oder tierischen Ursprungs, binden kann. Man verwendet eine unlösliche Adsorptionsmatrix, da in diesem Fall eine leichtere Abtrennung von der Probe möglich ist.

Gute Ergebnisse werden mit einer Adsorptionsmatrix auf Basis von Kohlenhydraten oder/und Polypeptiden erhalten. Bevorzugt ist eine Adsorptionsmatrix auf Basis von Kohlenhydraten, z.B. eine Adsorptionsmatrix, die Polysaccharide enthält. Besonders bevorzugt verwendet man eine Adsorptionsmatrix, die α- oder/und β-glykosidisch verknüpfte Kohlenhydrate enthält, z.B. Stärke, Cellulose, Glykogen oder/und andere biogene oder nichtbiogene Kohlenhydrate sowie Derivate oder Mischungen davon.

Am meisten bevorzugt ist die Verwendung von Mehlen, d.h. im wesentlichen einer Mischung von Cellulose, Stärke, Lipiden und Salzen oder Bestandteilen daraus. Als geeignet haben sich beispielsweise Mehle aus Getreide, Mais, Erbsen, Soja und Kartoffeln oder Bestandteile daraus bzw. Mischungen davon, erwiesen. Für einen Fachmann ist es selbstverständlich, daß neben den genannten Mehlsorten auch andere Mehlsorten bzw. Mischungen von mehreren Mehlsorten oder Bestandteilen daraus eingesetzt werden können. Am meisten bevorzugt ist die Verwendung von Kartoffelmehl oder Bestandteilen daraus. Ebenso bevorzugt sind Mischungen aus gereinigten Kohlenhydraten, z.B. Cellulose, und Mehlen, z.B. Kartoffelmehl.

Weiterhin bevorzugt ist die Verwendung einer Adsorptionsmatrix auf Kohlenhydratbasis zusammen mit löslichen Bestandteilen aus Mehlen, insbesondere aus einer oder mehreren der obengenannten Mehlsorten.

Die Menge, in der die Adsorptionsmatrix der biologischen Probe zugesetzt wird, hängt im wesentlichen von der Beschaffenheit der Probe, d.h. der Menge an Verunreinigungen, ab. Gute Ergebnisse wurden erhalten, wenn man die Adsorptionsmatrix in einem Gewichtsanteil von 0,05 : 1 bis 100 : 1 bezüglich der Nukleinsäuren enthaltenden Probe verwendet. Besonders bevorzugt wird die Adsorptionsmatrix in einer Menge von 0,1 : 1 bis 10 : 1 zugesetzt.

Die Nukleinsäuren enthaltende Probe, die durch das erfindungsgemäße Verfahren stabilisiert werden soll, stammt aus biologischen Materialien, die Nukleinsäuren abbauende bzw. enzymatische Reaktionen hemmende Verunreinigungen enthalten. Vorzugsweise stammt die Nukleinsäuren enthaltende Probe aus Fäkalien. Sie kann jedoch auch beispielsweise aus anderen Quellen, z.B. Geweben jeder Art, Knochenmark, humanen und tierischen Körperflüssigkeiten wie Blut, Serum, Plasma, Urin, Sperma, Cerebrospinalflüssigkeit, Sputum und Abstrichen, Pflanzen, Pflanzenteilen und -extrakten, z.B. -säften, Pilzen, Mikroorganismen wie Bakterien, fossilen oder mumifizierten Proben, Bodenproben, Klärschlamm, Abwässern und Lebensmitteln stammen. Als Verunreinigungen können beispielsweise Abbauprodukte von Hämoglobin, wie etwa Bilirubin und dessen Abbauprodukte, oder/und Gallensäuren oder Salze davon oder deren Abbauprodukte, aber auch andere Arten von Verunreinigungen enthalten sein.

Zur besseren Handhabung des Verfahrens hat es sich als günstig erwiesen, die Probe aus biologischen Materialien vor dem Zusatz der Adsorptionsmatrix in einer Pufferlösung aufzunehmen. Die Inkubation der Probe mit der Adsorptionsmatrix kann bei Raumtemperatur erfolgen. Die Inkubationsdauer kann in weiten Bereichen variiert werden. Nach der Inkubation kann die Adsorptionsmatrix z.B. durch Zentrifugation von der Probe abgetrennt werden. Alternativ kann die Probe direkt mit der Adsorptionsmatrix versetzt werden, z.B. bei flüssigen biologischen Proben. Weiterhin kann die Probe über eine Adsorptionsmatrix durch Zentrifugation, durch Anlegen eines Vakuums oder/und mittels der Schwerkraft geführt werden, wobei die Adsorptionsmatrix vorzugsweise dann abgefüllt in einer Säule vorliegt.

Die Behandlung mit der Adsorptionsmatrix führt zu einer signifikanten Stabilitätserhöhung der in der Probe enthaltenen Nukleinsäuren und bei einer anschließenden Isolierung der Nukleinsäuren zu einer besseren Reproduzierbarkeit. Dies gilt insbesondere, wenn sich an die Isolierung eine enzymatische Manipulation der Nukleinsäuren, z.B. eine Amplifikation oder/und eine Restriktionsspaltung anschließt. Besonders bevorzugt wird die Amplifikation, z.B. durch PCR (Polymerase Chain Reaction), LCR (Ligase Chain Reaction), NASBA (Nucleic Acid Base Specific Amplification) oder 3SR (Self Sustained Sequence Replication) durchgeführt.

Ein besonders bevorzugter Aspekt der vorliegenden Erfindung ist die Analyse, der Nachweis oder die Isolierung von Nukleinsäuren, insbesondere DNA, aus Stuhlproben. Durch das erfindungsgemäße Verfahren sind saubere und amplifizierbare Nukleinsäuren aus fäkalen Proben erhältlich, die zum Nachweis von Mutationen, insbesondere von tumorspezifischen DNA-Mutationen verwendet werden können.

Das erfindungsgemäße Verfahren besitzt große Bedeutung für die Tumordiagnostik, da es den spezifischen Nachweis nukleärer eukaryotischer Nukleinsäuren in Gegenwart von Verunreinigungen und großer Mengen an bakteriellen Nukleinsäuren ermöglicht.

Durch Analyse von Stuhl-DNA unter Anwendung des erfindungsgemäßen Verfahrens können Tumoren des Verdauungstrakts, insbesondere Pankreas- oder Darmtumoren, früher und genauer diagnostiziert werden. Diese Diagnose erfolgt beispielsweise durch Untersuchung von Onkogenen oder/und Tumorsuppressorgenen auf tumorspezifische DNA-Mutationen. Da Zellen von Tumoren des Verdauungstraktes laufend in den Stuhl abgeschilfert werden, ist eine Detektion von tumorspezifischen DNA-Mutationen im Stuhl durch das erfindungsgemäße Verfahren möglich. Das erfindungsgemäße Verfahren erlaubt außerdem das Monitoring von Therapien, die im Hinblick auf die Eliminierung eines Tumors eingeschlagen wurden, sowie die regelmäßige und verläßliche Durchführung von Tumorpräventionsuntersuchungen.

Im Gegensatz zum einzigen, aus dem Stand der Technik bekannten nicht-invasiven Routinetest auf kolorektale Tumoren, dem Test auf okkultes Blut im Stuhl, kommt es beim erfindungsgemäßen Verfahren nicht bzw. nur sehr selten zu falsch-positiven Ergebnissen. Darüber hinaus ermöglicht der Nachweis von Mutationen in Genen, die schon im Adenomstadium, d.h. in einem sehr frühen Stadium der Tumorprogression mutieren, eine deutlich frühere und spezifischere Diagnose als der Stuhl-Bluttest. Als geeignete Objekte der Mutationsanalysen können insbesondere das Tumorsupressorgen APC (Adenomatöse Polyposis Coli) (Fearon und Vogelstein (1990), Cell 61, 759-761) und das ras-Onkogen verwendet werden. Durch Mutationsanalysen dieser beiden Gene in DNA aus Stuhlproben können insbesondere Darmtumoren, z.B. Dickdarmtumoren, und Pankreastumoren erfaßt werden. Neben dem APC-Gen und dem ras-Gen können natürlich auch weitere tumorrelevante Gene für die Krebsdiagnose als Analyseobjekte dienen.

Abgesehen von tumorrelevanten Genen können auch nichttranslatierte repetitive Abschnitte des Genoms als Analyseobjekte in der Krebsdiagnose dienen. Diese sogenannten Mikrosatellitenabschnitte werden amplifiziert und das gelelektrophoretisch erhaltene Bandenmuster mit dem Bandenmuster von DNA aus gesundem Körpermaterial desselben Patienten verglichen. Unterschiedliche Bandenmuster können Hinweise auf das Vorhandensein eines Tumors liefern.

Ein weiteres Beispiel für die Anwendung des erfindungsgemäßen Verfahrens ist eine genaue Identifikation von Personen durch Untersuchung der aus Fäkalien oder Körpermaterial gereinigten Nukleinsäuren in der forensischen Analyse. Dazu werden repetitive polymorphe Abschnitte des Genoms amplifiziert und die Amplifikationsprodukte gelelektrophoretisch aufgetrennt. Durch einen Vergleich der erhaltenen Bandenmuster mit den Mustern von DNA anderer verdächtiger oder eng verwandter Personen kann dann die betreffende Person identifiziert werden.

Eine weitere wichtige Anwendung für die erfindungsgemäße Isolierung von DNA aus fäkalen Proben sind zoobiologische populationsgenetische, evolutionsgenetische und botanische Studien und Untersuchungen von Tieren und Pflanzen. Bisher scheitern derartige Untersuchungen sehr häufig an der Seltenheit einer Tierart und der geringen Wahrscheinlichkeit, die betreffenden Tiere an einem bestimmten Ort anzutreffen. Bei Kenntnis des ungefähren Aufenthaltsortes kann eine Analyse von zurückgelassenen Fäkalien durch das erfindungsgemäße Verfahren wichtige Hinweise auf den Verwandtschaftsgrad der Tiere, auf zurückgelegte Wanderungswege oder auf die Nahrungsgewohnheiten der Tiere liefern. Ebenso können aus der Analyse fäkaler Nukleinsäuren, z.B. durch Nachweis mikrobieller oder viraler Nukleinsäuren, wichtige diagnostische Informationen über Infektionen, z.B. bakterieller oder viraler Art abgeleitet werden.

Ein weiterer Gegenstand der vorliegenden Erfindung ist ein Reagenzienkit zur Stabilisierung und Reinigung von Nukleinsäuren aus biologischen Materialien, umfassend:
(a) einen zur Aufnahme einer Nukleinsäuren enthaltenden Probe geeigneten Puffer und
(b) eine unlösliche Adsorptionsmatrix zur Bindung von Verunreinigungen aus den biologischen Materialien, die derart beschaffen ist, daß sie Verunreinigungen, die zur Schädigung von Nukleinsäuren führen oder/und die Durchführung enzymatischer Reaktionen verhindern oder/und enzymatische Reaktionen hemmen, binden kann.

Die Adsorptionsmatrix kann in einer abgepackten portionierten Form, z.B. abgefüllt in einer Säule wie etwa einer zentrifugierbaren Minisäule, vorliegen.

Vorzugsweise enthält das Reagenzienkit zusätzliche Mittel zur Reinigung von Nukleinsäuren, die z.B. mineralische oder/und organische Träger sowie gegebenenfalls Lösungen, Hilfsstoffe oder/und Zubehör umfassen. Mineralische Bestandteile von Trägern können beispielsweise poröse oder nicht-poröse Metalloxide oder Metallmischoxide, z.B. Aluminiumoxid, Titandioxid oder Zirkoniumdioxid, Silicagele, Materialien auf Basis von Gläsern, z.B. modifizierte oder nicht-modifizierte Glaspartikel oder Glasmehl, Quarz, Zeolithe oder Mischungen von einer oder mehrerer der obengenannten Substanzen sein. Andererseits kann der Träger auch organische Bestandteile enthalten, die z.B. aus gegebenenfalls mit funktionellen Gruppen modifizierten Latexpartikeln, synthetischen Polymeren, wie etwa Polyethylen, Polypropylen, Polyvinylidenfluorid, insbesondere ultrahochmolekularem Polyethylen oder HD-Polyethylen, oder Mischungen von einer oder mehreren der zuvor genannten Substanzen ausgewählt werden.

Der Träger kann beispielsweise in Form von Partikeln mit einer mittleren Größe von 0,1 µm bis 1000 µm vorliegen. Bei Verwendung eines porösen Trägers ist eine mittlere Porengröße von 2 µm bis 1000 µm bevorzugt. Der Träger kann beispielsweise in Form loser Schüttungen von Partikeln, Filterschichten, z.B. aus Glas, Quarz oder Keramik, Membranen, z.B. Membranen, in denen ein Silicagel angeordnet ist, Fasern oder Geweben aus mineralischen Trägern, wie etwa Quarz oder Glaswolle sowie in Form von Latices oder Frittenmaterialien aus synthetischen Polymeren vorliegen.

Weiterhin kann das Reagenzienkit auch noch geeignete Lösungen, z.B. Waschlösungen oder Pufferlösungen zur Aufnahme der Probe enthalten. Ein zur Aufnahme einer Nukleinsäuren enthaltenden Probe geeigneter Puffer ist beispielsweise ein Puffersystem auf Basis von Tris-HCl pH 8,5-9,5, EDTA und gegebenenfalls NaCl. Ein besonders bevorzugter Puffer, insbesondere zur Aufnahme von Stuhlproben, enthält 500 mM Tris-HCl pH 9, 50 mM EDTA und 10 mM NaCl.

Außerdem kann das erfindungsgemäße Reagenzienkit auch Hilfsstoffe wie Enzyme und andere Mittel zur Manipulation von Nukleinsäuren enthalten, z.B. mindestens einen Amplifikationsprimer und zur Amplifikation von Nukleinsäuren geeignete Enzyme, z.B. eine Nukleinsäurepolymerase oder/und mindestens eine Restriktionsendonuklease.

Die Primer zur Amplifikation von Nukleinsäuren stammen zweckmäßigerweise aus den zu analysierenden Genen, d.h. beispielsweise aus Onkogenen, Tumorsupressorgenen oder/und Mikrosatellitenabschnitten. Zur Amplifikation von Nukleinsäuren geeignete Enzyme und Restriktionsendonukleasen sind bekannt und kommerziell erhältlich.

Weiterhin soll die vorliegende Erfindung durch das nachfolgende Beispiel erläutert werden.

### Beispiel 1

### Analyse von DNA aus Stuhlproben

Es wurden folgende Adsorptionsmatrizen getestet: Immobilisiertes Rinderserum-Albumin (RSA), Cellulose und Kartoffelstärke (alle von Sigma, München, DE) und Kartoffelmehl (Honig, Postbus 45, 1540 AA Koog a/d Zaan, NL), bei dem es sich im wesentlichen um eine unlösliche Mischung aus Cellulose, Stärke, Lipiden und Salzen handelt.

Menschliche Stuhlproben wurden gesammelt, eingefroren und bei - 80°C aufbewahrt. 200 mg Stuhl wurden in 600 µl Stuhl-Lösepuffer (SLP: 500 mM Tris-HCl pH 9,0, 50 mM EDTA, 10 mM NaCl) homogenisiert. Zu jeweils 1/4 des Homogenats wurden 200 µl SLP mit 100 mg der jeweiligen Adsorptionsmatrix gegeben. Die Mischung wurde heftig vermischt und zweimal bei 500 x g bzw. 13000 x g für jeweils 5 min zur Präzipitation von Bakterien und anderen Verunreinigungen zentrifugiert. Nach Behandlung des klaren Überstands mit Proteinase K in einer Konzentration von 2,5 mg/ml wurde die DNA unter Verwendung einer DNA-Spinsäule (Qiagen, Hilden, DE) gereinigt, die zur DNA-Reinigung aus Blut und Gewebe geeignet ist. Die Säulenbeladung und Waschschritte wurden wie vom Hersteller angegeben, durchgeführt.

Die DNA wurde dann aus der Spinsäule in einem Endvolumen von 150 µl destillierten Wasser eluiert und bis zur Verwendung bei - 20°C aufbewahrt. Die Ausbeute an chromosomaler DNA wurde durch Messung der Absorption bei 260 nm bestimmt.

Alle Präparationen zeigten vergleichbare Gesamt-DNA-Mengen von 15-20 µg. Auf einem analytischen Agarosegel waren keine Unterschiede zwischen der genomischen DNA aus Präparationen mit und ohne Adsorptionsmatrix zu erkennen. Durch Zugabe der Adsorptionsmatrix wurde auch keine Erhöhung in der Ausbeute der extrahierten chromosomalen DNA festgestellt.

Zum Test der Stabilität der isolierten Nukleinsäuren wurde die DNA nach einwöchiger Aufbewahrung untersucht. Die Ergebnisse sind in Tabelle 1 gezeigt. Die stabilsten DNA-Proben wurden nach Verwendung von Kartoffelmehl als Adsorptionsmatrix erhalten.

Für die Amplifizierung durch PCR wurden 3 µl der gereinigten chromosomalen DNA in einem Gesamtvolumen von 50 µl verwendet, das 10 mM Tris-HCl pH 8,3, 50 mM KCl, 1,5 mM/MgCl₂, 30 µM jeweils dATP, dCTP, dGTP und dTTP, 400 nM jedes Primers, 100 µg/ml RSA und 0,75 Einheiten Taq-Polymerase (AGS, Heidelberg, DE) enthielt.

Zur Sensitivitätsbesserung wurden Nested-PCR-Methoden (vgl. Jackson et al., (1991), in McPherson, N.J. Quirke, P. Taylor, G.R. (Hrsg.), PCR-A Practical Approach, Oxford University Press) unter Verwendung von Biotin-markierten Nested-Primern durchgeführt. Eine PCR von in Abwesenheit einer Adsorptionsmatrix gereinigten DNA-Proben war vollständig blockiert. Durch Zusatz von RSA, Cellulose oder Kartoffelstärke als Adsorptionsmatrix konnten teilweise reproduzierbare PCR-Ergebnisse erhalten werden (Tabelle 1).

Reproduzierbare PCR-Ergebnisse in allen zehn analysierten Proben wurden bei Verwendung von Kartoffelmehl als Adsorptionsmatrix erhalten. Die PCR-Fragmente waren zur Verwendung bei der Heteroduplexanalyse und auch zur direkten Sequenzierung geeignet. Hierzu erfolgte eine Präparation von einzelsträngiger DNA unter Verwendung von Streptavidin-gekoppelten Magnetbeads (Dynal, Hamburg, DE) gemäß den Angaben des Herstellers.

**TABELLE 1:**

| Eigenschaften von nukleärer DNA aus Stuhl | | |
|---|---|---|
| **Matrix** | **Verlust**^{**a**} | **PCR**^{**b**} |
| - | 80 % | 0 |
| RSA | 60 % | 3 |
| Cellulose | 60 % | 4 |
| Kartoffelstärke | 60 % | 4 |
| Kartoffelmehl | < 5 % | 10 |

| | | |
|---|---|---|
| ^{a} Der DNA-Verlust durch Abbau wurde nach Aufbewahrung für 1 Woche bei - 20°C durch analytische Agarosegelelektrophorese und spektrophotometrische Analyse gemessen. | | |
| ^{b} Es wurde eine PCR von DNA aus zehn verschiedenen Stuhlproben durchgeführt. Es ist die Anzahl von Proben angegeben, die durch PCR analysierbar waren. | | |

### Beispiel 2

### Reinigung von Stuhlproben

Verwendete Puffer:
- Puffer SLP:: (siehe Beispiel 1)
- Puffer A:: 5,6 M Guanidinium/HCl; 20% Tween 20
- Puffer B:: 10 mM Tris/HCl pH 7,5; 100 mM NaCl; 70% Ethanol
- Puffer C:: 10 mM Tris/HCl pH 9,0; 0,5 mM EDTA

Von einer gefrorenen Stuhlprobe wurden 3 g eingewogen und mit 2 ml Puffer SLP durch gründliches Vortexen gemischt. Anschließend wurde eine Säule mit einer 1:1 Mischung (w/w) aus Kartoffelmehl und Cellulose gefüllt und in ein 50 ml Zentrifugationsröhrchen gesteckt. Die in Puffer aufgenommene Probe wurde in diese Säule überführt und durch Zentrifugation bei 500 Upm für 5 Min von Verunreinigungen geklärt.

Zu 1,2 ml geklärter Probe wurden 0,125 ml Proteinase K-Stammlösung (1,785 mg/ml) und 1,2 ml Puffer A gegeben. Die Probe wurde durch Vortexen für 1 Min gemischt und anschließend für 10 Min bei 70°C inkubiert. Nach Zugabe von 1,3 ml absolutem Alkohol und gründlichem Mischen wurde die Lösung in eine Qia-AMP-Midisäule (Qiagen) transferiert und die Nukleinsäuren durch Zentrifugation auf einer Silikamatrix gebunden.

Die gebundenen Nukleinsäuren wurden durch zweimaliges Waschen mit 2,5 ml Puffer B gereinigt und mit 0,5 ml Puffer C von der Qia-AMP-Midisäule eluiert und zur weiteren Verwendung bei -20°C gelagert.

Bei einer wie in Beispiel 1 durchgeführten PCR an den gelagerten Proben konnten reproduzierbare Ergebnisse erhalten werden.

## Patentansprüche

1. Verfahren zur Reinigung, Stabilisierung oder/und Isolierung von Nukleinsäuren aus einer Probe aus biologischen Materialien,
**dadurch gekennzeichnet,**
**daß** man der Nukleinsäuren enthaltenden Probe eine unlösliche Adsorptionsmatrix zur Bindung von Verunreinigungen zusetzt, die derart beschaffen ist, daß sie Verunreinigungen, die zur Schädigung von Nukleinsäuren führen oder/und die Durchführung enzymatischer Reaktionen verhindern oder/und enzymatische Reaktionen hemmen, binden kann.

2. Verfahren nach Anspruch 1,
**dadurch gekennzeichnet,**
**daß** man eine Adsorptionsmatrix auf Kohlenhydratbasis verwendet.

3. Verfahren nach Anspruch 1 oder 2,
**dadurch gekennzeichnet,**
**daß** man eine Adsorptionsmatrix verwendet, die α- oder/ und β-glykosidisch verknüpfte Kohlenhydrate enthält.

4. Verfahren nach einem der Ansprüche 1-3,
**dadurch gekennzeichnet,**
**daß** man eine Adsorptionsmatrix verwendet, die Stärke, Cellulose, Glykogen oder/und andere biogene oder nichtbiogene Kohlenhydrate oder Mischungen davon enthält.

5. Verfahren nach einem der Ansprüche 1-4,
**dadurch gekennzeichnet,**
**daß** man als Adsorptionsmatrix ein Mehl aus Getreide, Erbsen, Mais, Soja, Kartoffeln oder Bestandteile daraus oder Mischungen davon verwendet.

6. Verfahren nach Anspruch 5,
**dadurch gekennzeichnet,**
**daß** man Kartoffelmehl oder Bestandteile daraus verwendet.

7. Verfahren nach einem der Ansprüche 1-6,
**dadurch gekennzeichnet,**
**daß** man eine Adsorptionsmatrix auf Kohlenhydratbasis zusammen mit löslichen Bestandteilen aus Mehlen verwendet.

8. Verfahren nach einem der Ansprüche 1-7,
**dadurch gekennzeichnet,**
**daß** man als Adsorptionsmatrix Mischungen aus gereinigten Kohlenhydraten oder/und Mehlen verwendet.

9. Verfahren nach einem der Ansprüche 1-8,
**dadurch gekennzeichnet,**
**daß** man als Adsorptionsmatrix Mischungen aus Cellulose und Kartoffelmehl verwendet.

10. Verfahren nach einem der Ansprüche 1-9,
**dadurch gekennzeichnet,**
**daß** man die Probe aus biologischen Materialien vor dem Zusatz der Adsorptionsmatrix in einer Pufferlösung aufnimmt.

11. Verfahren nach einem der Ansprüche 1-10,
**dadurch gekennzeichnet,**
**daß** man die Probe aus biologischen Materialien direkt mit der Adsorptionsmatrix versetzt.

12. Verfahren nach einem der Ansprüche 1-11,
**dadurch gekennzeichnet,**
**daß** sich an die Isolierung der Nukleinsäuren deren direkte Analyse anschließt.

13. Verfahren nach einem der Ansprüche 1-12,
**dadurch gekennzeichnet,**
**daß** sich an die Isolierung eine enzymatische Manipulation der Nukleinsäuren anschließt.

14. Verfahren nach Anspruch 13,
**dadurch gekennzeichnet,**
**daß** die enzymatische Manipulation der Nukleinsäuren durch PCR (Polymerase Chain Reaction), LCR (Ligase Chain Reaction), NASBA (Nucleic Acid Base Specific Amplification) oder 3SR (Self Sustained Sequence Replication) erfolgt.

15. Verwendung eines Verfahrens nach einem der Ansprüche 1-14 zur Analyse, zum Nachweis oder zur Isolierung von Nukleinsäuren aus Stuhlproben.

16. Verwendung nach Anspruch 15 zum Nachweis von DNA-Mutationen.

17. Verwendung nach Anspruch 15 oder 16 zur Analyse, zum Nachweis oder zur Isolierung nukleärer eukaryontischer Nukleinsäuren.

18. Verwendung nach Anspruch 17 zur Diagnostik von Tumoren des Verdauungstrakts, insbesondere von Pankreas- oder Darmtumoren.

19. Verwendung nach Anspruch 18,
**dadurch gekennzeichnet,**
**daß** man Onkogene, Tumorsuppressorgene oder/und Mikrosatellitenabschnitte untersucht.

20. Verwendung nach Anspruch 17 zur Untersuchung von Tieren und Pflanzen.

21. Verwendung nach Anspruch 15 zum Nachweis mikrobieller oder viraler Nukleinsäuren.

22. Verwendung nach Anspruch 21 zur Diagnostik bakterieller und viraler Infektionen.

23. Verwendung eines Verfahrens nach einem der Ansprüche 1-14 zum Verwandtschaftsnachweis und zur forensischen Identifikation von individuellen Personen.

24. Reagenzienkit zur Reinigung und Stabilisierung von Nukleinsäuren aus biologischen Materialien, umfassend:
(a) einen zur Aufnahme einer Nukleinsäuren enthaltenden Probe geeigneten Puffer und
(b) eine unlösliche Adsorptionsmatrix zur Bindung von Verunreinigungen aus den biologischen Materialien, die derart beschaffen ist, daß sie Verunreinigungen, die zur Schädigung von Nukleinsäuren führen oder/und die Durchführung enzymatischer Reaktionen verhindern oder/und enzymatische Reaktionen hemmen, binden kann.

25. Reagenzienkit nach Anspruch 24, zusätzlich umfassend Mittel zur weiteren Reinigung von Nukleinsäuren.

26. Reagenzienkit nach Anspruch 25,
**dadurch gekennzeichnet,**
**daß** die Mittel zur weiteren Reinigung von Nukleinsäuren mineralische oder/und organische Träger sowie gegebenenfalls Lösungen, Hilfsstoffe oder/und Zubehör umfassen.

27. Reagenzienkit nach einem der Ansprüche 24-26,
**dadurch gekennzeichnet,**
**daß** die Adsorptionsmatrix in einer Säule abgefüllt ist.

## Claims

1. Method for purifying, stabilizing or/and isolating nucleic acids from a specimen of biological materials,
**characterized in that**
an insoluble adsorption matrix is added to the specimen containing nucleic acids to bind contaminants wherein the adsorption matrix is made in such a way that it can bind contaminants which damage nucleic acids or/and prevent enzymatic reactions from being carried out or/and inhibit enzymatic reactions.

2. Method as claimed in claim 1,
**characterized in that**
an adsorption matrix based on carbohydrates is used.

3. Method as claimed in claim 1 or 2,
**characterized in that**
an adsorption matrix is used which contains carbohydrates with α- or/and β-glycosidic linkages.

4. Method as claimed in one of the claims 1 - 3,
**characterized in that**
an adsorption matrix is used which contains starch, cellulose, glycogen or/and other biogenic or nonbiogenic carbohydrates or mixtures thereof.

5. Method as claimed in one of the claims 1 - 4,
**characterized in that**
a flour made from grain, peas, maize, soya, potatoes or components or mixtures thereof is used as the adsorption matrix.

6. Method as claimed in claim 5,
**characterized in that**
potato flour or components thereof are used.

7. Method as claimed in one of the claims 1 - 6,
**characterized in that**
an adsorption matrix based on carbohydrates is used together with soluble flour components.

8. Method as claimed in one of the claims 1 - 7,
**characterized in that**
mixtures of purified carbohydrates or/and flours are used as the adsorption matrix.

9. Method as claimed in one of the claims 1 - 8,
**characterized in that**
mixtures of cellulose and potato flour are used as the adsorption matrix.

10. Method as claimed in one of the claims 1 - 9,
**characterized in that**
the specimen of biological materials is taken up in a buffer solution prior to adding the adsorption matrix.

11. Method as claimed in one of the claims 1 - 10,
**characterized in that**
the adsorption matrix is added directly to the specimen of biological materials.

12. Method as claimed in one of the claims 1 - 11,
**characterized in that**
the nucleic acids are analysed directly after their isolation.

13. Method as claimed in one of the claims 1 - 12,
**characterized in that**
the isolation is followed by an enzymatic manipulation of the nucleic acids.

14. Method as claimed in claim 13,
**characterized in that**
the enzymatic manipulation of the nucleic acids is carried out by means of PCR (Polymerase Chain Reaction), LCR (Ligase Chain Reaction), NASBA (Nucleic Acid Base Specific Amplification) or 3SR (Self Sustained Sequence Replication).

15. Use of a method as claimed in one of the claims 1-14 for the analysis, detection or isolation of nucleic acids from stool specimens.

16. Use as claimed in claim 15 for the detection of DNA mutations.

17. Use as claimed in claim 15 or 16 for the analysis, detection or isolation of nuclear eukaryotic nucleic acids.

18. Use as claimed in claim 17 for the diagnosis of tumours of the digestive tract, in particular of pancreatic and intestinal tumours.

19. Use as claimed in claim 18,
**characterized in that**
oncogenes , tumour suppressor genes or/and microsatellite sections are examined.

20. Use as claimed in claim 17 for the examination of animals and plants.

21. Use as claimed in claim 15 for detecting microbial or viral nucleic acids.

22. Use as claimed in claim 21 for diagnosing bacterial and viral infections.

23. Use of a method as claimed in one of the claims 1-14 for proving relationships and for the forensic identification of individual persons.

24. Reagent kit for the purification and stabilization of nucleic acids from biological materials comprising:
(a) a buffer suitable for taking up a specimen containing nucleic acids and
(b) an insoluble adsorption matrix for binding contaminants from the biological materials, which are made in such a way that it can bind contaminants which damage nucleic acids or/and prevent enzymatic reactions from being carried out or/and inhibit enzymatic reactions.

25. Reagent kit as claimed in claim 24, additionally comprising means to further purify nucleic acids.

26. Reagent kit as claimed in claim 25,
**characterized in that**
the means to further purify nucleic acids comprise mineral or/and organic support materials and optionally solutions, auxiliary substances or/and accessories.

27. Reagent kit as claimed in one of the claims 24-26,
**characterized in that**
the adsorption matrix is packed in a column.

## Revendications

1. Procédé pour la purification, la stabilisation et/ou l'isolement d'acides nucléiques d'un échantillon de matériaux biologiques, **caractérisé en ce que** l'on ajoute à l'échantillon contenant des acides nucléiques une matrice d'adsorption insoluble pour la liaison des impuretés qui est constituée de telle manière qu'elle peut lier les impuretés qui conduisent à une détérioration des acides nucléiques et/ou qui empêchent la conduite de réactions enzymatiques et/ou qui inhibent les réactions enzymatiques.

2. Procédé selon la revendication 1 **caractérisé en ce que** l'on utilise une matrice d'adsorption à base glucidique.

3. Procédé selon la revendication 1 ou 2 **caractérisé en ce que** l'on utilise une matrice d'adsorption qui contient des glucides liés par des liaisons glycosidiques α et/ou β.

4. Procédé selon l'une des revendications 1 à 3 **caractérisé en ce que** l'on utilise une matrice d'adsorption qui contient de l'amidon, de la cellulose, du glycogène et/ou d'autres glucides biogènes ou non biogènes ou des mélanges de ceux-ci.

5. Procédé selon l'une des revendications 1 à 4 **caractérisé en ce que** l'on utilise comme matrice d'adsorption une farine de céréales, de pois, de maïs, de soja, de pommes de terre ou de constituants ou mélanges de ceux-ci.

6. Procédé selon la revendication 5 **caractérisé en ce que** l'on utilise de la farine de pommes de terre ou des constituants de celle-ci.

7. Procédé selon l'une des revendications 1 à 6 **caractérisé en ce que** l'on utilise une matrice d'adsorption à base glucidique en même temps que des constituants solubles de farines.

8. Procédé selon l'une des revendications 1 à 7 **caractérisé en ce que** l'on utilise comme matrice d'adsorption des mélanges de glucides purifiés et/ou de farines.

9. Procédé selon l'une des revendications 1 à 8 **caractérisé en ce que** l'on utilise comme matrice d'adsorption des mélanges de cellulose et de farine de pommes de terre.

10. Procédé selon l'une des revendications 1 à 9 **caractérisé en ce que** l'on introduit l'échantillon de matériaux biologiques dans une solution tampon avant d'ajouter la matrice d'adsorption.

11. Procédé selon l'une des revendications 1 à 10 **caractérisé en ce que** l'on ajoute la matrice d'adsorption directement à l'échantillon de matériaux biologiques.

12. Procédé selon l'une des revendications 1 à 11 **caractérisé en ce que** l'analyse directe des acides nucléiques suit leur isolement.

13. Procédé selon l'une des revendications 1 à 12 **caractérisé en ce qu'**une manipulation enzymatique des acides nucléiques suit l'isolement.

14. Procédé selon la revendication 13 **caractérisé en ce que** la manipulation enzymatique des acides nucléiques a lieu par PCR (Polymerase Chain Reaction), LCR (Ligase Chain Reaction), NASBA (Nucleic Acid Base Specific Amplification) ou 3SR (Self Sustained Sequence Replication).

15. Utilisation d'un procédé selon l'une des revendications 1 à 14 pour l'analyse, pour la mise en évidence ou pour l'isolement d'acides nucléiques d'échantillons de selles.

16. Utilisation selon la revendication 15 pour la mise en évidence de mutations d'ADN.

17. Utilisation selon la revendication 15 ou 16 pour l'analyse, pour la mise en évidence ou pour l'isolement d'acides nucléiques eucaryotes nucléaires.

18. Utilisation selon la revendication 17 pour le diagnostic de tumeurs des voies digestives, en particulier de tumeurs du pancréas ou de l'intestin.

19. Utilisation selon la revendication 18 **caractérisée en ce que** l'on étudie des oncogènes, des gènes suppresseurs de tumeurs et/ou des segments de microsatellites.

20. Utilisation selon la revendication 17 pour l'étude d'animaux et de végétaux.

21. Utilisation selon la revendication 15 pour la mise en évidence d'acides nucléiques microbiens ou viraux.

22. Utilisation selon la revendication 21 pour le diagnostic d'infections bactériennes et virales.

23. Utilisation d'un procédé selon l'une des revendications 1 à 14 pour la mise en évidence de parenté et pour l'identification légale de personnes individuelles.

24. Kit de réactifs pour la purification et la stabilisation d'acides nucléiques de matériaux biologiques, comprenant :
(c) un tampon approprié pour recevoir un échantillon contenant des acides nucléiques et
(d) une matrice d'adsorption insoluble pour la liaison d'impuretés provenant des matériaux biologiques, qui est constituée de telle manière qu'elle peut lier des impuretés qui conduisent à une détérioration des acides nucléiques et/ou qui empêchent la conduite de réactions enzymatiques et/ou qui inhibent les réactions enzymatiques.

25. Kit de réactifs selon la revendication 24 comprenant en outre des moyens pour la purification supplémentaire d'acides nucléiques.

26. Kit de réactifs selon la revendication 25 **caractérisé en ce que** les moyens pour la purification supplémentaire d'acides nucléiques comprennent des supports minéraux et/ou organiques et éventuellement des solutions, des adjuvants et/ou des accessoires.

27. Kit de réactifs selon l'une des revendications 24 à 26 **caractérisé en ce que** la matrice d'adsorption est contenue dans une colonne.
